# EUROPEAN PATENT APPLICATION

(11) **EP 4 394 014 A1**
(43) Date of publication of application: **03.07.2024**
(21) Application number: 22860404.7
(22) Date of filing: 19.08.2022
(51) Int. Cl.: C09K 8/584, C08G 65/28, C07C 213/04, C07C 303/32

(54) **HYDROCARBYL TETRALIN POLYETHERSULFONATE, PREPARATION METHOD THEREFOR AND USE THEREOF**

(30) Priority: 23.08.2021 CN 202110967009
(71) Applicant: China Petroleum & Chemical Corporation, Beijing 100728 (CN); Shanghai Research Institute of Petrochemical Technology, SINOPEC, Shanghai 201208 (CN)
(72) Inventor: BAO, Xinning, Shanghai 201208 (CN); LI, Yingcheng, Shanghai 201208 (CN); ZHANG, Weidong, Shanghai 201208 (CN); JIN, Jun, Shanghai 201208 (CN); MENG, Yong, Shanghai 201208 (CN); ZHANG, Li, Shanghai 201208 (CN)
(74) Representative: karo IP
(86) International application number: PCT/CN2022/113592
(87) International publication number: WO 2023/025058

(57) **Abstract**

The present invention provides a hydrocarbyl tetralin polyethersulfonate, preparation method therefor and use thereof. The hydrocarbyl tetralin polyethersulfonate is shown by formula (I-0),

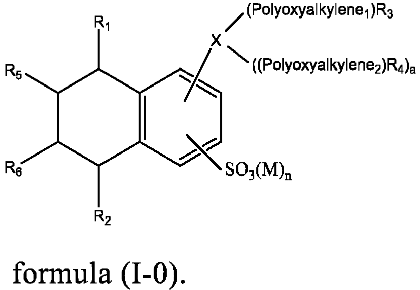

The hydrocarbyl tetralin polyethersulfonate of the present invention has a high surface and interface activity, can be used in an oil reservoir exploitation, especially in a high-temperature and high-salt oil reservoir exploitation, and has improved the crude oil recovery.

## Description

### TECHNICAL FIELD

The present invention belongs to the technical field of oil-displacing agents, and particularly relates to a hydrocarbyl tetralin polyethersulfonate, a preparation method therefor and a use thereof.

### BACKGROUND TECHNOLOGY

The method of using surfactants as oil displacing agents to improve crude oil recovery is called "surfactant flooding". The surfactant flooding is a widely used, markedly effective and suitable method for improving tertiary oil recovery. There are a variety of surfactants, mainly including anionic, cationic, non-ionic and zwitterionic surfactants. Surfactants improve an oil displacement efficiency by changing the interfacial tension and the emulsification between the oil displacement system and the crude oil and increasing the solubility of the crude oil. Surfactants play a key role for improving oil recovery in the chemical flooding. Developing a highly efficient, inexpensive, temperature resistant and salt tolerant surfactant is of great significance for promoting the development of chemical combination flooding technology.

The commonly used surfactant products mainly include anionic surfactants such as petroleum sulfonates, alkylbenzene sulfonates, olefin sulfonates, etc. The above surfactants have a certain effect on the conventional oil reservoirs with a temperature of 80°C and a total dissolved solid of 30,000 mg/L or less. At present, a great many surfactants have been successfully applied in the ordinary oil reservoirs (Class I and Class II oil reservoirs), but 60% or more of the remaining oil reserves are harsh oil reservoirs that are difficult to exploit, such as the high temperature, high salinity, high water content, high clay, low permeability, and heavy oil reservoirs. The above surfactants are less efficient or even ineffective due to their low activity and poor salt tolerance.

### CONTENTS OF THE INVENTION

To solve at least one of the above problems in the prior art, the present invention provides a hydrocarbyl tetralin polyethersulfonate, a preparation method therefor and a use thereof. The hydrocarbyl tetralin polyethersulfonate of the present invention has a high surface and interface activity and can improve the recovery of crude oil.

The object of the present invention is realized through the following technical solutions.

In a first aspect, the present invention provides a hydrocarbyl tetralin polyethersulfonate shown by formula (I-0), 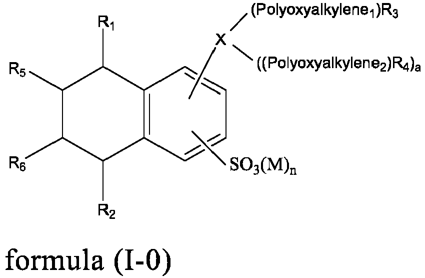 wherein:
R₁, R₂, R₅ and R₆ are each independently H or C₁-C₃₀ hydrocarbyl, provided that at least one of R₁, R₂, R₅ and R₆ is not H;
R₃, R₄ are independently selected from H, C₁-C₁₀ hydrocarbyl, C₁-C₁₀ hydrocarbon carbonyl, C₁-C₁₀ hydrocarbon sulfonic group, C₁-C₁₀ hydrocarbon alcohol sulfonic group, C₁-C₁₀ hydrocarbon carboxylic group or -SO₃(M)ₙ;
X is selected from N and O atoms, provided that a=1 when group X is N, and a=0 when group Y is O;
-(Polyoxyalkylene₁)- is one or more of -(PO)ₓ₁-, -(EO)_{y1}- and -(BO)_{z1}-, and -(Polyoxyalkylene₂)- is one or more of -(PO)ₓ₂-, -(EO)_{y2}- and -(BO)_{z2}-; provided that x1+x2=0∼30, y1+y2=1~30, z1+z2=0∼30;
M is selected from any one of alkali metal ion and alkaline earth metal ion. When M is an alkali metal ion, n is 1, and when M is an alkaline earth metal ion, n is 0.5;
wherein the hydrocarbon moiety of R₁₋R₆ is not substituted, or is substituted or inserted by a heteroatom selected from O, S, and N.

In an embodiment, in the hydrocarbyl tetralin polyethersulfonate shown by formula (I-0), the hydrocarbyl moieties of R₁, R₂, R₅ and R₆ are selected from a linear or branched C₁-C₃₀ alkyl, preferably C₁-C₁₆ alkyl; C₁-C₃₀ alkoxy, preferably C₁-C₁₆ alkoxy; C₃-C₃₀ cycloalkyl, preferably C₃-C₁₄ cycloalkyl; C₃-C₃₀ heterocyclic group, preferably C₃-C₁₄ heterocyclic group, such as imidazole, pyrrole, pyridine; phenyl unsubstituted or substituted by one or more identical or different C₁-C₄ alkyl; five- or six-membered heteroaromatic group containing 1-4 N or S atoms.

Without being limited by any known theory, the inventors have found that for the use of oil displacing agents, especially in terms of the recovery, for example for the interfacial tension, in the hydrocarbyl tetralin polyethersulfonate shown by formula (I-0), it is particularly advantageous that all groups R₁, R₂, R₅ and R₆ are not H, and it is particularly preferred that at least one group of them is alkyl; furthermore, the inventors have also found that it is relatively disadvantageous when the number of C atoms in R₁, R₂, R₅ and R₆ is too high or too low. Therefore, it is preferred that at least part or all of R₁, R₂, R₅ and R₆ have an appropriate number of C atoms. Correspondingly, in an embodiment, in the hydrocarbyl tetralin polyethersulfonate shown by formula (I-0), preferably the sum of the carbon atom numbers of R₁, R₂, R₅ and R₆ is 8-16, and/or preferably R₁, R₂, R₅ and R₆ are each independently selected from alkyl.

In an embodiment, in the hydrocarbyl tetralin polyethersulfonate shown by formula (I-0), R₅ and R₆ are H, X is N.

The hydrocarbyl tetralin polyethersulfonate shown by formula (I-0) of the present invention can be particularly used for the applications for oil displacing agents, e.g. as a surfactant; therefore, the present invention further provides a surfactant composition comprising the hydrocarbyl tetralin polyethersulfonate shown by formula (I-0). Furthermore, the present invention also provides an oil displacing agent composition comprising the hydrocarbyl tetralin polyethersulfonate shown by formula (1-0).

In an embodiment, the hydrocarbyl tetralin polyethersulfonate shown by formula (I-0) in the present invention has the following formula (I): 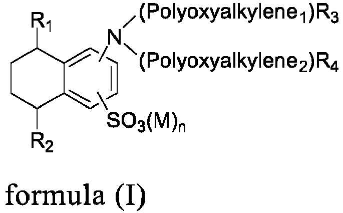 wherein R₁ is C₆-C₃₀ hydrocarbyl; R₂ is H or C₁-C₃₀ hydrocarbyl; R₃ and R₄ are each independently selected from H, C₁-C₁₀ hydrocarbyl, C₁-C₁₀ hydrocarbon carbonyl, C₁-C₁₀ hydrocarbon sulfonic group, C₁-C₁₀ hydrocarbon alcohol sulfonic group, C₁-C₁₀ hydrocarbon carboxylic group or -SO₃(M)ₙ; -(Polyoxyalkylene)₁- is one or more of -(PO)ₓ₁-, -(EO)_{y1}- and -(BO)_{z1}-; -(Polyoxyalkylene)₂- is one or more of -(PO)ₓ₂-, -(EO)_{y2}- and - (BO)_{z2}-, x1+x2=0-30, y1+y2=1-30, z1+z2=0-30; M is selected from any one of alkali metal ion and alkaline earth metal ion; when M is an alkali metal ion, n is 1, and when M is an alkaline earth metal ion, n is 0.5.

In the present invention, "PO" represents the repeating unit -CH₂-CH(CH₃)-O-; "EO" represents the repeating unit -CH₂-CH₂-O-; and "BO" represents the repeating unit -CH₂-CH(CH₂CH₃)-O-.

According to the hydrocarbyl tetralin polyethersulfonate provided by the present invention, when -(Polyoxyalkylene₁)- is more than one of - (PO)ₓ₁-, -(EO)_{y1}- and -(BO)_{z1}-, there is no special requirement for their connection sequence in the present invention. For instance, for the purpose of the present invention, they may preferably be -(BO)_{z1}-(PO)ₓ₁-(EO)_{y1}-, - (BO)_{z1}-(EO)_{y1}-(PO)ₓ₁-, -(PO)ₓ₁-(EO)_{y1}-(BO)_{z1}-, -(PO)ₓ₁-(BO)_{z1}-(EO)_{y1}-, - (EO)_{y1}-(BO)_{z1}-(PO)ₓ₁-, -(EO)_{y1}-(PO)ₓ₁-(BO)_{z1}-. Similarly, when - (Polyoxyalkylene₂)- is more than one of -(PO)ₓ₂-, -(EO)_{y2}- and -(BO)_{z2}-, there is no special requirement for their connection sequence in the present invention. For instance, for the purpose of the present invention, they may, for example, be -(BO)_{z2}-(PO)ₓ₂-(EO)_{y2}-, -(BO)_{z2}-(EO)_{y2}-(PO)ₓ₂-, -(PO)ₓ₂-(EO)_{y2}-(BO)_{z2}-, -(PO)ₓ₂-(BO)_{z2}-(EO)_{y2}-, -(EO)_{y2}-(BO)_{z2}-(PO)ₓ₂-, -(EO)_{y2}-(PO)_{xz}-(BO)_{z2}-. Without being limited by any known theory, for the purpose of the present invention, especially for the use of oil displacing agents, the preferred connection sequence may be -BO_{z1}-POₓ₁-EO_{y1}- or -BO_{z2}-POₓ₂-EO_{y2}-.

According to an embodiment provided by the present invention, in formula (I), R₁ may be alkyl, alkenyl or aryl.

According to an embodiment provided by the present invention, in formula (I), R₁ may be C₈-C₁₆ hydrocarbyl, preferably C₈-C₁₂ hydrocarbyl.

In some preferred embodiments, R₁ is C₈-C₁₆ alkyl; and in some preferred embodiments, R₁ is C₈-C₁₂ alkyl. For instance, examples of such alkyl include but are not limited to: octyl, nonyl, decyl, undecyl and dodecyl.

According to an embodiment provided by the present invention, in formula (I), R₂ is H or C₁-C₃₀ alkyl.

In some embodiments, R₂ is H or C₁-C₁₂ alkyl; and R₂ is H or C₁-C₈ alkyl in some embodiments. For instance, examples of alkyl of such R₂ include but are not limited to: methyl, ethyl, propyl, butyl, pentyl, hexyl, heptyl and octyl.

According to an embodiment provided by the present invention, in formula (I), R₃, R₄ are each independently selected from H, C₁-C₁₀ alkyl, C₁-C₁₀ alkyl carbonyl, C₁₋C₁₀ alkyl sulfonic group, C₁-C₁₀ alkyl alcohol sulfonic group, C₁-C₁₀ alkyl carboxylic group or -SO₃(M)ₙ.

In some embodiments, in formula (I), R₃, R₄ are independently selected from H, C₁-C₆ alkyl or -SO₃(M)ₙ.

According to an embodiment provided by the present invention, as R₃, examples of C₁-C₆ alkyl include but are not limited to: methyl, ethyl, propyl, butyl, pentyl and hexyl.

In some embodiments, in formula (I), R₃ is H, -CH₃, -CH₂CH₃ or - SO₃(M)ₙ.

According to an embodiment provided by the present invention, in formula (I), both x1 and y1 are not simultaneously 0; both x2 and y2 are not simultaneously 0.

In some embodiments, in formula (I), z1 and z2 are zero, x1+x2=0-12, y1+y2=4-20; in some embodiments, z1 and z2 are zero, x1+x2=0-8, y1+y2=4-12; and in some embodiments, z1 and z2 are zero, x1+x2=0-4, y1+y2=4-8.

According to an embodiment provided by the present invention, in formula (I), z1 and z2 are zero, x1+x2+y1+y2=1-50, preferably 5-20, more preferably 5-15.

Without being limited by any known theory, for the purpose of the present invention, especially for the use of oil displacing agents, e.g. based on the considerations of interfacial tension, PO and EO units may be present simultaneously; correspondingly, in an exemplary embodiment, z1 and z2 are zero, x1+x2=1-6, y1+y2=4-8.

Without being limited by any known theory, for the purpose of the present invention, especially for the use of oil displacing agents, e.g. based on the considerations of hydrophilic and oleophilic balance, PO, EO and BO units may be present simultaneously; correspondingly, in an exemplary embodiment, x1+x2=1-6, y1+y2=4-8, z1+z2=1-6.

According to an embodiment provided by the present invention, for M, examples of alkali metal ions include but are not limited to: lithium ion, sodium ion and potassium ion. Similarly, for M, examples of alkaline earth metal ions include but are not limited to: beryllium ion, magnesium ion and calcium ion.

In some preferred embodiments, M is selected from any one of sodium ion, potassium ion, calcium ion and magnesium ion.

According to an embodiment provided by the present invention, the present invention does not have special requirements for the positions of - SO₃(M)ₙ and 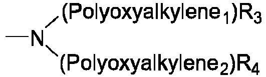 (referred to as an amino polyether group in the present application) on the tetralin ring.

According to an embodiment provided by the present invention, for example, -SO₃(M)ₙ can be located at position 5, 6, 7 or 8 of the tetralin ring; whereas the amino polyether group can be located at position 5, 6, 7 or 8 of the tetralin ring, which is different from the position of -SO₃(M)ₙ. In an embodiment, the amino polyether group is located at position 5 or 8 of the tetralin ring, preferably at position 5 of the tetralin ring; and/or the -SO₃(M)ₙ is located at position 6 or 7 of the tetralin ring. In an embodiment, preferably the amino polyether group and the -SO₃(M)ₙ are in a meta position with each other.

In another aspect, the present invention provides a method for preparing the hydrocarbyl tetralin polyethersulfonate of formula (I-0) or formula (I), wherein, taking the situation in which X is N as an example, the preparation method comprises the following steps: S100. reacting the hydrocarbyl tetralin shown by formula (II-0) or formula (II) with a nitration reagent to produce hydrocarbyl nitrotetralin;

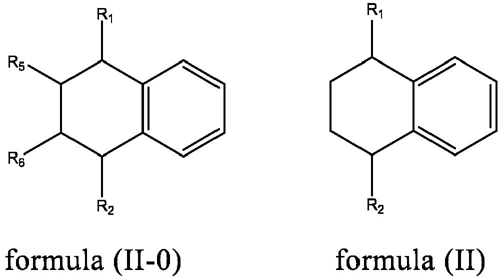

In formula (II-0) or formula (II), R₁, R₂, R₅ and R₆ are each as described above, for example are independently H or C₁-C₃₀ hydrocarbyl, provided that at least one of R₁, R₂, R₅ and R₆ is not H. In an embodiment, R₁ is C₆-C₃₀ hydrocarbyl, and R₂ is H or C₁-C₃₀ hydrocarbyl;
S200. hydrotreating the hydrocarbyl nitrotetralin obtained in step S100 to obtain alkyl tetralin amine;
S300. reacting the hydrocarbyl tetralin amine obtained in step S200 with one or more of epoxyethane, epoxypropane, and epoxybutane to obtain hydrocarbyl tetralin amino polyether;
S400. reacting the hydrocarbyl tetralin amino polyether obtained in step S300 with a sulfonation reagent to obtain a sulfonated product which is hydrolyzed to obtain the hydrocarbyl tetralin polyethersulfonate of formula (I-0) or formula (I).

Correspondingly, it can be prepared similarly when X is O in formula (I-0). The major differences lie in: (1) sulfonating hydrocarbyl tetralin as the raw material with sulfuric acid to generate tetralin sulfonic acid, neutralizing with sulphurous acid, and then alkali melting with caustic soda to produce it; or (2) carrying out a halogenation reaction via hydrocarbyl tetralin and then hydrolyzing at a high temperature to obtain it.

According to the preparation method provided by the present invention, there are no special requirements for the nitration reagent in the present invention, and any known nitration reagent in the art can be used; however, for the purpose of oil displacing agents, it is preferred in some embodiments that the nitration reagent in step S100 is at least one of nitric acid and dinitrogen pentoxide, or a mixed acid composed of at least one of nitric acid and dinitrogen pentoxide and at least one selected from concentrated sulfuric acid, glacial acetic acid, acetic anhydride and phosphorus pentoxide.

In the present invention, when the mixed acid is used as the nitration reagent, it is preferred that the molar ratio of at least one of nitric acid and dinitrogen pentoxide to at least one selected from concentrated sulfuric acid, glacial acetic acid, acetic anhydride and phosphorus pentoxide is 2-6:1.

According to an embodiment of the preparation method provided in the present invention, in the step S100, the molar ratio of the hydrocarbyl tetralin to the nitration reagent is 1:1-3; the reaction temperature is preferably 0-80°C; the reaction time is preferably 1-10 hours. In some embodiments, the reaction temperature is 20-60°C, the reaction time is 1-3 hours in the step S100.

According to an embodiment of the preparation method provided in the present invention, the step S200 includes: reacting the hydrocarbyl nitrotetralin obtained in step S100 with hydrogen in the presence of a hydrogenation catalyst to obtain hydrocarbyl tetralin amine.

Examples of hydrogenation catalysts suitable for use in the present invention include but are not limited to: palladium carbon catalysts and raney nickel catalysts.

In some embodiments, the amount of the hydrogenation catalyst is 0.01% to 10%, preferably 0.1% to 5%, of the weight of the hydrocarbyl nitrotetralin.

According to an embodiment of the preparation method provided in the present invention, the hydrotreatment in step S200 is carried out at a temperature of 20-150°C and a pressure of less than 4 MPa. In some embodiments, the hydrotreatment in step S200 is carried out at a temperature of 50-70°C and a pressure of 1-3 MPa.

According to an embodiment of the preparation method provided in the present invention, in step S300, the amounts of epoxypropane and epoxyethane can be determined based on the target structure of the hydrocarbyl tetralin polyethersulfonate.

In some embodiments, in step S300, the molar ratio of the epoxypropane to the hydrocarbyl tetralin amine is 0-30:1; the molar ratio of the epoxyethane to the hydrocarbyl tetralin amine is 1-30:1; the molar ratio of the epoxybutane to the hydrocarbyl tetralin amine is 0-30:1.

In some embodiments, the amount of the epoxybutane is zero; the molar ratio of the epoxypropane to the hydrocarbyl tetralin amine is 0-12:1 and the molar ratio of the epoxyethane to the hydrocarbyl tetralin amine is 4-20:1; in some embodiments, the molar ratio of the epoxypropane to the hydrocarbyl tetralin amine is 0-8:1 and the molar ratio of the epoxyethane to the hydrocarbyl tetralin amine is 4-12:1; and in some embodiments, the molar ratio of the epoxypropane to the hydrocarbyl tetralin amine is 0-4:1 and the molar ratio of the epoxyethane to the hydrocarbyl tetralin amine is 4-8:1.

In some preferred embodiments, the amount of the epoxybutane in step S300 is zero, and the molar ratio of the total amount of the epoxypropane and the epoxyethane to the hydrocarbyl tetralin amine is 1-50:1, preferably 5-20:1, more preferably 5-15:1.

According to the preparation method provided by the present invention, the reaction of the hydrocarbyl tetralin amine with epoxypropane and optionally epoxypropane in step S300 is carried out in the presence of an alkaline catalyst.

Examples of alkaline catalysts suitable for use in the present invention include but are not limited to: alkali metals, alkali metal hydroxides, alkaline earth metal hydroxides, alkali metal alcoholates, and alkali metal oxides. In some embodiments, the alkaline catalyst is selected from at least one of alkali metal hydroxide and alkaline earth metal hydroxide; and in some embodiments, the alkaline catalyst is sodium hydroxide and/or potassium hydroxide.

In some embodiments, the amount of the alkaline catalyst is 0.005-2% by weight, preferably 0.05-1% by weight, based on the weight of the hydrocarbyl tetralin amine.

According to the preparation method provided by the present invention, the reaction temperature is 135-200°C and the pressure is 0-5 MPa in step S300. In some embodiments, the reaction temperature is 140-180°C and the pressure is 0-3 MPa in step S300, and in some embodiments, the reaction temperature is 140-160°C and the pressure is 0-0.4 MPa in step S300.

According to the preparation method provided by the present invention, step S300 further includes: performing a removal treatment at a temperature of 80-110°C after the reaction of the hydrocarbyl tetralin amine with epoxypropane and optionally epoxypropane. In the present invention, the removal treatment can be carried out by vacuum or a nitrogen bubbling.

According to the preparation method provided by the present invention, the sulfonation reagent in step S400 is at least one of concentrated sulfuric acid, fuming sulfuric acid or sulfur trioxide.

According to the preparation method provided by the present invention, the step S400 includes:
S401. reacting the hydrocarbyl tetralin amino polyether with the sulfonation reagent in a molar ratio of 1:1-5 at a temperature of 20-80°C for 0.5-10 hours to obtain a sulfonated product;
S402. adjusting the pH value of the sulfonated product obtained in step S401 to 10-14 and hydrolyzing for 0.5-5 hours to obtain the hydrocarbyl tetralin polyethersulfonate.

In step S402 of the present invention, an alkali can be used to adjust the pH value of the sulfonated product. In the present invention, the alkali is selected from at least one of alkali metal hydroxide and alkaline earth metal hydroxide. In some specific embodiments, the alkali is sodium hydroxide and/or potassium hydroxide.

In another aspect, the present invention provides the hydrocarbyl tetralin polyethersulfonate prepared by the preparation method.

In a further aspect, the present invention also provides an oil displacing agent composition, wherein the oil displacing agent composition comprises the hydrocarbyl tetralin polyethersulfonate and water.

According to the oil displacing agent composition provided by the present invention, the weight ratio of the hydrocarbyl tetralin polyethersulfonate to water is 1:(50-2000), preferably 1:(100-1000).

In the present invention, there is a wide range of selection for the types of water. The water can be either deionized water or mineral-containing water. In some embodiments, the total mineral content of the mineral-containing water is 0-150000 mg/L; and 10000-100000 mg/L in some embodiments.

Examples of mineral-containing water suitable for use in the present invention include but are not limited to: mineralized water, oilfield injection water, formation water, seawater, rainwater and river water.

In some specific embodiments, for the sake of the construction convenience and the water resource conservation etc., oilfield injection water is preferred, for example the injection water of the Shengli Oilfield used in the examples of the present invention, the constitution of which is shown in Table 1.

According to the oil displacing agent composition provided by the present invention, to enhance the oil displacement effect, the oil displacing agent composition of the present invention may also include common additives in the art, such as at least one of polyacrylamide, C1-C6 small molecule alcohol, dimethyl sulfoxide (DMSO), diethanolamine and cetyltrimethylammonium chloride (CTAC) etc..

The above raw materials in the present invention can all be self-made or commercially available, and there is no special limitation on this in the present invention.

In another aspect, the present invention provides the use of said hydrocarbyl tetralin polyethersulfonate or said oil displacing agent composition in an oil reservoir exploitation, especially in the high temperature and high salinity oil reservoir exploitation.

The present invention has the following advantages: the hydrocarbyl tetralin polyethersulfonate of the present invention is a new type of anionic non-ionic sulfonate surfactant, has advantages of strong temperature resistance and salt tolerance and low adsorption, etc., has a good affinity with crude oil, has a high surface and interface activity, and can form a relatively low interfacial tension on the oil-water interface. It can be used for enhancing oil recovery in chemical flooding, especially in the high-temperature and high-salinity oil reservoirs, as can effectively improve the recovery of crude oil and has a broad application prospect and a practical significance.

### MODE OF CARRYING OUT THE INVENTION

The present invention is further illustrated below in combination with specific examples which, however, do not form any limitation to the present invention.

If there is no special restriction on the raw materials used in the examples and the comparative examples, they are all disclosed in the prior art, for example they are able to be directly purchased or prepared according to the preparation methods disclosed in the prior art.

### Example 1

### 1. Synthesis of 1-octyltetralin-5-amino polyoxypropylene (30) polyoxyethylene (16) ether-7-sodium sulfonate

a) 1.0 mol of 1-octyltetralin was added to a reactor equipped with a condensing device and a stirring device, and 2.1 mol of fuming nitric acid was added dropwise. The reaction temperature was controlled to 55±5°C. After the dropwise addition was completed, the reaction was continued for 2 hours. Let it stand to separate the solution, thereby obtaining 1-octyl-5-nitrotetralin.
b) 1-octyl-5-nitrotetralin was added to a high pressure reactor, and a palladium carbon catalyst with a specification of 10% was added according to 0.5% of the weight of the reaction substrate, and the reactor was sealed. It was filled with nitrogen gas for replacement for 5 times, followed by a hydrogen gas replacement for 5 times. The temperature was raised to 60°C and hydrogen began to be added. The system pressure was controlled at 3 MPa, and the reaction lasted for 6 hours to obtain 1-octyltetralin-5-amine.
c) 1-octyltetralin-5-amine and 1% by mass of sodium hydroxide were added to a reactor equipped with a condensing device, a stirring device and a gas disperser, heated to 85°C while nitrogen gas was introduced, and stirred to react for 1 hour. A vacuum system was turned on, and the reaction was heated to 90°C, vacuum dehydrated for 1 hour, then purged with nitrogen gas for 4 times to remove air from the system. Then the reaction temperature of the system was adjusted to 150°C, and epoxypropane and epoxyethane were introduced slowly in sequence, and the pressure was controlled to < 0.40 MPa to carry out an etherification reaction; after the reaction, the system was purged with nitrogen gas, neutralized after cooling, and dehydrated to obtain 1-octyltetralin-5-amino polyoxypropylene (30) polyoxyethylene (16) ether.
d) The octyltetralin amino polyoxypropylene (30) polyoxyethylene (16) ether synthesized in step c) was added to a reactor equipped with a condensing device, a dropwise addition device and a stirring device, and 20% fuming sulfuric acid was added dropwise in a molar ratio of 1:3. The reaction temperature was controlled to 50°C. After the dropwise addition was completed, the reaction was continued for 1 hour, and the excess acid was removed by water washing and extraction. The pH of the organic phase was adjusted to 9 by adding sodium hydroxide, thereby obtaining 0.75 mol of 1-octyltetralin-5-amino polyoxypropylene (30) polyoxyethylene (16) ether-7-sodium sulfonate.

### 2. Performance evaluation

### Formulation of an oil displacing agent composition:

1 part by weight of 1-octyltetralin-5-amino polyoxypropylene (30) polyoxyethylene (16) ether-7-sodium sulfonate was mixed with 1000 parts by weight of the injection water from the Zhongyuan Oilfield to obtain an oil displacing agent composition, which was used for an interfacial tension evaluation and an oil displacement experiment. The constitution of the used injection water from the Zhongyuan Oilfield is shown in Table 1. For the convenience of comparison, the constitution of the oil displacing agent composition is listed in Table 2.

### Interfacial tension evaluation:

The interfacial tension between the above oil displacing agent composition and the dehydrated crude oil from the Zhongyuan Oilfield was measured using a TX-500C Spinning Drop Interfacial Tensiometer produced by the University of Texas of the United States at 80°C and a rotational speed of 4500 rpm. The results are shown in Table 3.

### Example 2

### 1. Synthesis of 1-dodecyltetralin-5-amino polyoxyethylene (6) ether-7-sodium sulfonate

a) 1.0 mol of 1-dodecyltetralin was added to a reactor equipped with a condensing device and a stirring device, and 1.1 equivalents of 65% nitric acid and 50 g of 98% concentrated sulfuric acid were added dropwise according to a molar ratio. The reaction temperature was controlled to 20°C. After the dropwise addition was completed, the reaction was continued for 1 hour. Let it stand to separate the solution, thereby obtaining 1-dodecyl-5-nitrotetralin.
b) 1-dodecyl-5-nitrotetralin was added to a high pressure reactor, and a palladium carbon with a specification of 10% was added according to 0.5% of the weight of the reaction substrate, and the reactor was sealed. It was filled with nitrogen gas for replacement for 5 times, followed by hydrogen gas replacement for 5 times. The temperature was raised to 60°C and hydrogen began to be added. The system pressure was controlled at 3 MPa, and the reaction lasted for 6 hours to obtain 1-dodecyl-5-tetralinamine.
c) 1-dodecyl-5-tetralinamine and 1% by mass of sodium hydroxide were added to a reactor equipped with a condensing device, a stirring device and a gas disperser, heated to 85°C while nitrogen gas was introduced, and stirred to react for 1 hour. A vacuum system was turned on, and the reaction was heated to 90°C, vacuum dehydrated for 1 hour, then purged with nitrogen gas for 4 times to remove air from the system. Then the reaction temperature of the system was adjusted to 150°C, and epoxyethane was introduced slowly, and the pressure was controlled to ≤ 0.40 MPa to carry out an etherification reaction; after the reaction, the system was purged with nitrogen gas, neutralized after cooling, and dehydrated to obtain 1-dodecyltetralin-5-amino polyoxyethylene (6) ether.
d) The 1-dodecyltetralin-5-amino polyoxyethylene (6) ether synthesized in step c) was added to a reactor equipped with a condensing device, a dropwise addition device and a stirring device, and 3 equivalents of 20% fuming sulfuric acid were added dropwise according to a molar ratio. The reaction temperature was controlled to 50°C. After the dropwise addition was completed, the reaction was continued for 1 hour. Then sodium hydroxide was added to adjust the pH to 13, followed by a hydrolysis reaction for 2 hours to obtain 0.78 mol of 1-dodecyltetralin-5-amino polyoxyethylene (6) ether-7-sodium sulfonate.

### 2. Performance evaluation

According to the method in Example 1, the oil displacing agent composition was formulated and the interfacial tension was measured. For the convenience of comparison, the constitution of the oil displacing agent composition is listed in Table 2, and the evaluation results are shown in Table 3.

### Example 3

### 1. Synthesis of 1-dodecyl-4-octyltetralin-5-amino polyoxypropylene (4) polyoxyethylene (8) ether-7-sodium sulfonate

a) 1.0 mol of 1-dodecyl-4-octyltetralin was added to a reactor equipped with a condensing device and a stirring device, and 1.2 equivalents of 65% nitric acid and 0.3 equivalents of 98% concentrated sulfuric acid were added dropwise according to a molar ratio. The reaction temperature was controlled to 20°C. After the dropwise addition was completed, the reaction was continued for 1 hour. Let it stand to separate the solution, thereby obtaining 1-dodecyl-4-octyl-5-nitrotetralin.
b) 1-dodecyl-4-octyl-5-nitrotetralin was added to a high pressure reactor, and a palladium carbon with a specification of 10% was added according to 1.0% of the weight of the reaction substrate, and the reactor was sealed. It was filled with nitrogen gas for replacement for 5 times, followed by hydrogen gas replacement for 5 times. The temperature was raised to 60°C and hydrogen began to be added. The system pressure was controlled at 3 MPa, and the reaction lasted for 6 hours to obtain 1-dodecyl-4-octyltetralin-5-amine.
c) 1-dodecyl-4-octyltetralin-5-amine and 1% by mass of sodium hydroxide were added to a reactor equipped with a condensing device, a stirring device and a gas disperser, heated to 85°C while nitrogen gas was introduced, and stirred to react for 1 hour. A vacuum system was turned on, and the reaction was heated to 90°C, vacuum dehydrated for 1 hour, then purged with nitrogen gas for 4 times to remove air from the system. Then the reaction temperature of the system was adjusted to 150°C, and epoxypropane and epoxyethane were introduced slowly in sequence, and the pressure was controlled to ≤ 0.40 MPa to carry out an etherification reaction; after the reaction, the system was purged with nitrogen gas, neutralized after cooling, and dehydrated to obtain 1-dodecyl-4-octyltetralin-5-amino polyoxypropylene (4) polyoxyethylene (8) ether.
d) The 1-dodecyl-4-octyltetralin-5-amino polyoxypropylene (4) polyoxyethylene (8) ether synthesized in step c) was added to a reactor equipped with a condensing device, a dropwise addition device and a stirring device, and 4 equivalents of 50% fuming sulfuric acid were added dropwise according to a molar ratio. The reaction temperature was controlled to 20°C. After the dropwise addition was completed, the reaction was continued for 1 hour. Then sodium hydroxide was added to adjust the pH to 13, followed by hydrolysis reaction for 2 hours to obtain 0.83 mol of 1-dodecyl-4-octyltetralin-5-amino polyoxypropylene (4) polyoxyethylene (8) ether-7-sodium sulfonate.

### 2. Performance evaluation

According to the method in Example 1, the oil displacing agent composition was formulated and the interfacial tension was measured. For the convenience of comparison, the constitution of the oil displacing agent composition is listed in Table 2, and the evaluation results are shown in Table 3.

### Example 4

### 1. Synthesis of 1-triacontyltetralin-5-amino polyoxypropylene (10) polyoxyethylene (20) ether-7-sodium sulfonate

a) 1.0 mol of 1-triacontyltetralin was added to a reactor equipped with a condensing device and a stirring device, and 1.1 equivalents of 65% nitric acid and 0.2 equivalents of 98% concentrated sulfuric acid were added dropwise according to a molar ratio. The reaction temperature was controlled to 20°C. After the dropwise addition was completed, the reaction was continued for 1 hour. Let it stand to separate the solution, thereby obtaining 1-triacontyl-5-nitrotetralin.
b) 1-triacontyl-5-nitrotetralin was added to a high pressure reactor, and a palladium carbon with a specification of 10% was added according to 5.0% of the weight of the reaction substrate, and the reactor was sealed. It was filled with nitrogen gas for replacement for 5 times, followed by hydrogen gas replacement for 5 times. The temperature was raised to 60°C and hydrogen began to be added. The system pressure was controlled at 3 MPa, and the reaction lasted for 6 hours to obtain 1-triacontyltetralin-5-amine.
c) 1-triacontyltetralin-5-amine and 1% by mass of sodium hydroxide based on the reaction substrate were added to a reactor equipped with a condensing device, a stirring device and a gas disperser, heated to 85°C while nitrogen gas was introduced, and stirred to react for 1 hour. A vacuum system was turned on, and the reaction was heated to 90°C, vacuum dehydrated for 1 hour, then purged with nitrogen gas for 4 times to remove air from the system. Then the reaction temperature of the system was adjusted to 150°C, and epoxypropane and epoxyethane were introduced slowly in sequence, and the pressure was controlled to < 0.40 MPa to carry out an etherification reaction; after the reaction, the system was purged with nitrogen gas, neutralized after cooling, and dehydrated to obtain 1-triacontyltetralin-5-amino polyoxypropylene (10) polyoxyethylene (20) ether.
d) The 1-triacontyltetralin-5-amino polyoxypropylene (10) polyoxyethylene (20) ether synthesized in step c) was added to a reactor equipped with a condensing device, a dropwise addition device and a stirring device, and 5 equivalents of 98% fuming sulfuric acid were added dropwise according to a molar ratio. The reaction temperature was controlled to 50°C. After the dropwise addition was completed, the reaction was continued for 1 hour. Then sodium hydroxide was added to adjust the pH to 13, followed by hydrolysis reaction for 2 hours to obtain 0.79 mol of 1-triacontyltetralin-5-amino polyoxypropylene (10) polyoxyethylene (20) ether-7-sodium sulfonate.

### 2. Performance evaluation

According to the method in Example 1, the oil displacing agent composition was formulated and the interfacial tension was measured. For the convenience of comparison, the constitution of the oil displacing agent composition is listed in Table 2, and the evaluation results are shown in Table 3.

### Example 5

The oil displacing agent composition was formulated and the interfacial tension was measured according to the method in Example 1 using the surfactant synthesized in Example 3. For the convenience of comparison, the constitution of the oil displacing agent composition is listed in Table 2, and the evaluation results are shown in Table 3.

### Example 6

### 1. Synthesis of 1-ethyl-2-butyl-3-propyl-4-ethyltetralin-5-amino polyoxybutene (2) polyoxypropylene (2) polyoxyethylene (6) ether-7-sodium sulfonate

a) 1.0 mol of 1-ethyl-2-butyl-3-propyl-4-ethyltetralin was added to a reactor equipped with a condensing device and a stirring device, and 1.05 equivalents of 65% nitric acid and 0.2 equivalents of 98% concentrated sulfuric acid were added dropwise according to a molar ratio. The reaction temperature was controlled to 30°C. After the dropwise addition was completed, the reaction was continued for 1 hour. Let it stand to separate the solution, thereby obtaining 1-ethyl-2-butyl-3-propyl-4-ethyl-5-nitrotetralin.
b) 1-ethyl-2-butyl-3-propyl-4-ethyl-5-nitrotetralin was added to a high pressure reactor, and a palladium carbon with a specification of 10% was added according to 0.2% of the weight of the reaction substrate, and the reactor was sealed. It was filled with nitrogen gas for replacement for 5 times, followed by hydrogen gas replacement for 5 times. The temperature was raised to 60°C and hydrogen began to be added. The system pressure was controlled at 1-3 MPa, and the reaction lasted for 6 hours to obtain 1-ethyl-2-butyl-3-propyl-4-ethyl-5-nitrotetralin amine.
c) 1-ethyl-2-butyl-3-propyl-4-ethyl-5-nitrotetralin amine and 0.5% by mass of sodium hydroxide were added to a reactor equipped with a condensing device, a stirring device and a gas disperser, heated to 85°C while nitrogen gas was introduced, and stirred to react for 1 hour. A vacuum system was turned on, and the reaction was heated to 90°C, vacuum dehydrated for 0.5 hours, then purged with nitrogen gas for 4 times to remove air from the system. Then the reaction temperature of the system was adjusted to 150°C, and epoxybutane, epoxypropane and epoxyethane were introduced slowly in sequence, and the pressure was controlled to < 0.40 MPa to carry out an etherification reaction; after the reaction, the system was purged with nitrogen gas, neutralized after cooling, and dehydrated to obtain 1-ethyl-2-butyl-3-propyl-4-ethyltetralin-5-amino polyoxybutene (2) polyoxypropylene (2) polyoxyethylene (6) ether.
d) The 1-ethyl-2-butyl-3-propyl-4-ethyltetralin-5-amino polyoxybutene (2) polyoxypropylene (2) polyoxyethylene (6) ether synthesized in step c) was dissolved in dichloroethane. The reaction temperature was controlled to 30°C. Sulfonation was carried out by introducing sulfur trioxide, and the reaction lasted for 0.5 hours. Then sodium hydroxide was added to adjust the pH to 12, followed by hydrolysis reaction at 80°C for 2 hours to obtain 0.81 mol of 1-ethyl-2-butyl-3-propyl-4-ethyltetralin-5-amino polyoxybutene (2) polyoxypropylene (2) polyoxyethylene (6) ether-7-sodium sulfonate.

### 2. Performance evaluation

According to the method in Example 1, the oil displacing agent composition was formulated and the interfacial tension was measured. For the convenience of comparison, the constitution of the oil displacing agent composition is listed in Table 2, and the evaluation results are shown in Table 3.

### Example 7

### 1. Synthesis of 1-butoxy-2-methyl-3-propyltetralin-5-amino polyoxybutene (3) polyoxypropylene (4) polyoxyethylene (8) ether-7-sodium sulfonate

a) 1.0 mol of 1-butoxy-2-methyl-3-propyltetralin was added to a reactor equipped with a condensing device and a stirring device, and 1.02 equivalents of nitric acid and 0.2 equivalents of acetic anhydride were added dropwise according to a molar ratio. The reaction temperature was controlled to 30°C. After the dropwise addition was completed, the reaction was continued for 1 hour. Let it stand to separate the solution, thereby obtaining 1-butoxy-2-methyl-3-propyl-5-nitrotetralin.
b) 1-butoxy-2-methyl-3-propyl-5-nitrotetralin was added to a high pressure reactor, and a palladium carbon with a specification of 10% was added according to 0.1% of the weight of the reaction substrate, and the reactor was sealed. It was filled with nitrogen gas for replacement for 5 times, followed by hydrogen gas replacement for 5 times. The temperature was raised to 80°C and hydrogen began to be added. The system pressure was controlled at 1-3 MPa, and the reaction lasted for 6 hours to obtain 1-butoxy-2-methyl-3-propyl-5-nitrotetralin amine.
c) 1-butoxy-2-methyl-3-propyl-5-nitrotetralin amine and 0.5% by mass of sodium hydroxide were added to a reactor equipped with a condensing device, a stirring device and a gas disperser, heated to 85°C while nitrogen gas was introduced, and stirred to react for 1 hour. A vacuum system was turned on, and the reaction was heated to 90°C, vacuum dehydrated for 0.5 hours, then purged with nitrogen gas for 4 times to remove air from the system. Then the reaction temperature of the system was adjusted to 150°C, and epoxybutane, epoxypropane and epoxyethane were introduced slowly in sequence, and the pressure was controlled to < 0.50 MPa to carry out an etherification reaction; after the reaction, the system was purged with nitrogen gas, neutralized after cooling, and dehydrated to obtain 1-butoxy-2-methyl-3-propyltetralin-5-amino polyoxybutene (3) polyoxypropylene (4) polyoxyethylene (8) ether.
d) The 1-butoxy-2-methyl-3-propyltetralin-5-amino polyoxybutene (3) polyoxypropylene (4) polyoxyethylene (8) ether synthesized in step c) was dissolved in dichloroethane. The reaction temperature was controlled to 30°C. Sulfonation was carried out by introducing sulfur trioxide, and the reaction lasted for 0.5 hours. Then sodium hydroxide was added to adjust the pH to 12, followed by hydrolysis reaction at 80°C for 2 hours to obtain 0.83 mol of 1-butoxy-2-methyl-3-propyltetralin-5-amino polyoxybutene (3) polyoxypropylene (4) polyoxyethylene (8) ether-7-sodium sulfonate.

### 2. Performance evaluation

According to the method in Example 1, the oil displacing agent composition was formulated and the interfacial tension was measured. For the convenience of comparison, the constitution of the oil displacing agent composition is listed in Table 2, and the evaluation results are shown in Table 3.

**Table 1 constitution of the injection water from the Zhongyuan Oilfield**

| item, mg/L | | | | | | | |
|---|---|---|---|---|---|---|---|
| Na⁺+K⁺ | Mg²⁺ | Ca²⁺ | Cl⁻ | SO₄²⁻ | HCO₃⁻ | CO₃²⁻ | TDS |
| 24719 | 2871 | 592 | 37101 | 13939 | 217 | 24719 | 79439 |

**Table 2 constitutions of the oil displacing agent compositions in Examples 1-7**

| Examples | surfactant | | injection water from the Zhongyuan Oilfield, parts by weight |
|---|---|---|---|
| | 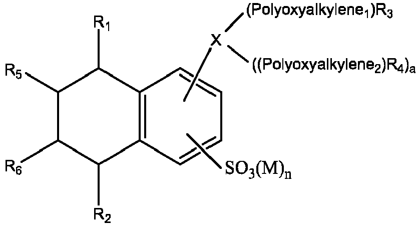 | | |
| | structural parameters | parts by weight | |
| 1 | R₁=C₈, R₂=H, R₃=-SO₃Na, R₄=-SO₃Na, R₅=H, R₆=H, n=1, x1+x2=30, y1+y2=16, z1+z2=0, M=Na, X=N | 1 | 1000 |
| 2 | R₁=C₁₂, R₂=H, R₃=H, R₄=H, R₅=H, R₆=H, n=1, x1+x2=0, y1+y2=6, z1+z2=0, M=Na, X=N | 1 | 300 |
| 3 | R₁=C₁₂, R₂=C₈, R₃=H, R₄=H, R₅=H, R₆=H, n=1, x1+x2=4, y1+y2=8, z1+z2=0, M=Na, X=N | 1 | 300 |
| 4 | R₁=C₃₀, R₂=H, R₃=H, R₄=H, R₅=H, R₆=H, n=1, x1+x2=10, y1+y2=20, z1+z2=0, M=Na, X=N | 1 | 500 |
| 5 | R₁=C₁₂, R₂=C₈, R₃=H, R₄=H, R₅=H, R₆=H, n=1, x1+x2=4, y1+y2=8, z1+z2=0, M=Na, X=N | 1 | 100 |
| 6 | R₁=C₂, R₂=C₂, R₃=H, R₄=H, R₅=C₄, R₆=C₃, n=1, x1+x2=2, y1+y2=6, z1+z2=2, M=Na, X=N | 1 | 200 |
| 7 | R₁=C₂H₁₀O, R₂=H, R₃=H, R₄= H, R₅=C₁, R₆=C₃, n=1, x1+x2=4, y1+y2=8, z1+z2=3, M=Na, X=N | 1 | 200 |

**Table 3 interfacial tension performance of the oil displacing agent compositions in Examples 1-7**

| Examples | interfacial tension (mN/m) |
|---|---|
| 1 | 0.028 |
| 2 | 0.0035 |
| 3 | 0.0079 |
| 4 | 0.015 |
| 5 | 0.0013 |
| 6 | 0.0011 |
| 7 | 0.0021 |

### Oil displacement effect test

According to the physical simulation oil displacement effect test of the composite oil displacement system in the SY/T6424-2000 composite oil displacement system performance testing method, a simulation oil displacement experiment was conducted on a core with a length of 30 cm, a diameter of 2.5 cm and a permeability of 1.5 m² at 80°C. First, the injection water from the Zhongyuan Oilfield was used for waterflooding to a water content of 98%. After the water flooding was completed, 0.3 pv (pore volume of the core) of the above oil displacing agent was injected, followed by waterflooding to a water content of 98% to calculate the increase in the crude oil recovery.

The oil displacing agents prepared in Example 3 and Example 5 were evaluated through oil displacement experiments according to the above method, and the results showed an increase in the crude oil recovery of 7.8% and 9.7%, respectively.

### Comparative Example 1

Sodium petroleum sulfonate (Shengli Oilfield) was used to replace the 1-dodecyl-4-octyltetralin-5-amino polyoxypropylene (4) polyoxyethylene (8) ether-7-sodium sulfonate surfactant in Example 3 to formulate an oil displacing agent composition, and the interfacial tension was measured. The interfacial tension between the oil displacing agent composition in Comparative Example 1 and the crude oil from the Zhongyuan Oilfield was 0.23 mN/m.

The above oil displacement effect test was used to measure the increase in the oil recovery of the oil displacing agent composition in Comparative Example 1, and the crude oil recovery was increased by 2.7%.

### Comparative Example 2

The following method was used to synthesize 1-dodecyl-4-octylnaphthalene-5-amino polyoxypropylene (4) polyoxyethylene (8) ether-7-sodium sulfonate.
a) 1.0 mol of 1-dodecyl-4-octylnaphthalene was added to a reactor equipped with a condensing device and a stirring device, and 1.1 equivalents of 65% nitric acid and 0.2 equivalents of 98% concentrated sulfuric acid were added dropwise according to a molar ratio. The reaction temperature was controlled to 30°C. After the dropwise addition was completed, the reaction was continued for 1 hour. Let it stand to separate the solution, thereby obtaining 1-dodecyl-4-octyl-5-nitronaphthalene.
b) 1-dodecyl-4-octyl-5-nitronaphthalene was added to a high pressure reactor, and a palladium carbon with a specification of 10% was added according to 0.5% of the weight of the reaction substrate, and the reactor was sealed. It was filled with nitrogen gas for replacement for 5 times, followed by hydrogen gas replacement for 5 times. The temperature was raised to 80°C and hydrogen began to be added. The system pressure was controlled at 3 MPa, and the reaction lasted for 5 hours to obtain 1-dodecyl-4-octylnaphthalene-5-amine.
c) 1-dodecyl-4-octylnaphthalene-5-amine and 1% by mass of sodium hydroxide were added to a reactor equipped with a condensing device, a stirring device and a gas disperser, heated to 85°C while nitrogen gas was introduced, and stirred to react for 0.5 hours. A vacuum system was turned on, and the reaction was heated to 90°C, vacuum dehydrated for 0.5 hours, then purged with nitrogen gas for 4 times to remove air from the system. Then the reaction temperature of the system was adjusted to 140°C, and epoxypropane and epoxyethane were introduced slowly in sequence, and the pressure was controlled to ≤ 0.50 MPa to carry out an etherification reaction; after the reaction, the system was purged with nitrogen gas, neutralized after cooling, and dehydrated to obtain 1-dodecyl-4-octylnaphthalene-5-amino polyoxypropylene (4) polyoxyethylene (8) ether.
d) The 1-dodecyl-4-octylnaphthalene-5-amino polyoxypropylene (4) polyoxyethylene (8) ether synthesized in step c) was added to a reactor equipped with a condensing device, a dropwise addition device and a stirring device, and 4 equivalents of 50% fuming sulfuric acid were added dropwise according to a molar ratio. The reaction temperature was controlled to 30°C. After the dropwise addition was completed, the reaction was continued for 1 hour. Then sodium hydroxide was added to adjust the pH to 12, followed by hydrolysis reaction at 80°C for 2 hours to obtain 0.79 mol of 1-dodecyl-4-octylnaphthalene-5-amino polyoxypropylene (4) polyoxyethylene (8) ether-7-sodium sulfonate.

1-dodecyl-4-octylnaphthalene-5-amino polyoxypropylene (4) polyoxyethylene (8) ether-7-sodium sulfonate was used to replace the 1-dodecyl-4-octyltetralin-5-amino polyoxypropylene (4) polyoxyethylene (8) ether-7-sodium sulfonate surfactant in Example 3 to formulate an oil displacing agent composition, and the interfacial tension was measured. The interfacial tension between the oil displacing agent composition in Comparative Example 2 and the crude oil from the Zhongyuan Oilfield is 0.012 mN/m.

The above oil displacement effect test was used to measure the increase in the oil recovery of the oil displacing agent composition in Comparative Example 2, and the crude oil recovery was increased by 4.6%.

Therefore, the hydrocarbyl tetralin polyethersulfonate of the present invention has a high surface and interface activity and can improve the recovery of crude oil.

For any values mentioned in the present invention, if there is only a two-unit interval between any lowest value and any highest value, it includes all the values that increase by one unit each time from the lowest value to the highest value. For example, if it is declared that the amount of a component or the value of process variables such as temperature, pressure, time, etc. is 50-90, it means in the present specification that the values such as 51-89, 52-88..., 69-71, and 70-71 are specifically listed. For non-integer values, it is appropriate to consider using 0.1, 0.01, 0.001 or 0.0001 as one unit. These are only some specially specified examples. In the present application, all possible combinations of the values between the lowest value and the highest value listed are considered to have been disclosed in a similar manner.

It should be noted that the above examples are only used to explain the present invention and do not form any limitation to the present invention. The present invention is described by referring to typical examples, but it should be understood that the words used therein are descriptive and explanatory vocabulary, rather than restrictive vocabulary. The present invention may be amended within the scopes of the claims of the present invention according to the stipulations, and may be revised without deviating from the scope and spirit of the present invention. Although the present invention described herein relates to specific methods, materials, and examples, it does not mean that the present invention is limited to the specific examples disclosed herein. On the contrary, the present invention can be extended to all other methods and uses with the same function.

## Claims

1. A hydrocarbyl tetralin polyethersulfonate shown by formula (I-0), 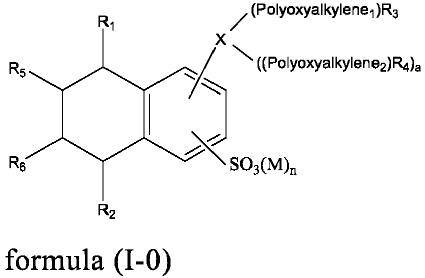 wherein:
R₁, R₂, R₅ and R₆ are each independently H or C₁-C₃₀ hydrocarbyl, provided that at least one of R₁, R₂, R₅ and R₆ is not H;
R₃, R₄ are independently selected from H, C₁-C₁₀ hydrocarbyl, C₁-C₁₀ hydrocarbon carbonyl, C₁-C₁₀ hydrocarbon sulfonic group, C₁-C₁₀ hydrocarbon alcohol sulfonic group, C₁-C₁₀ hydrocarbon carboxylic group or -SO₃(M)ₙ;
X is selected from N and O atoms, provided that a=1 when group X is N, and a=0 when group X is O;
-(Polyoxyalkylene₁)- is a group formed by connecting one or more of - (PO)ₓ₁-, -(EO)_{y1}- and -(BO)_{z1}-, and -(Polyoxyalkylene₂)- is a group formed by connecting one or more of -(PO)ₓ₂-, -(EO)_{y2}- and -(BO)_{z2}-; provided that x1+x2=0∼30, y1+y2=1∼30, z1+z2=0∼30;
M is selected from any one of alkali metal ion and alkaline earth metal ion; when M is an alkali metal ion, n is 1, and when M is an alkaline earth metal ion, n is 0.5;
wherein the hydrocarbyl moiety of R₁₋R₆ is not substituted, or is substituted or inserted by a heteroatom selected from O, S and N.

2. The hydrocarbyl tetralin polyethersulfonate according to claim 1, **characterized in that** the hydrocarbyl moieties of R₁, R₂, R₅ and R₆ are selected from a linear or branched C₁-C₃₀ alkyl, preferably C₁-C₁₆ alkyl; C₁-C₃₀ alkoxy, preferably C₁-C₁₆ alkoxy; C₃-C₃₀ cycloalkyl, preferably C₃-C₁₄ cycloalkyl; C₃-C₃₀ heterocyclic group, preferably C₃-C₁₄ heterocyclic group; phenyl unsubstituted or substituted by one or more identical or different C₁-C₄ alkyl; five- or six-membered heteroaromatic group containing 1-4 N or S atoms.

3. The hydrocarbyl tetralin polyethersulfonate according to claim 2, **characterized in that** the sum of the carbon atom numbers of R₁, R₂, R₅ and R₆ is 8-16, preferably R₁, R₂, R₅ and R₆ are each independently selected from alkyl.

4. The hydrocarbyl tetralin polyethersulfonate according to any one of claims 1-3, **characterized in that** R₅ and R₆ are H and X is N.

5. The hydrocarbyl tetralin polyethersulfonate according to claim 4, having formula (I), 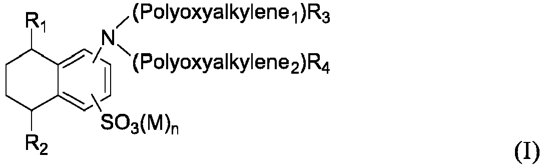 wherein R₁ is C₆-C₃₀ hydrocarbyl; R₂ is H or C₁-C₃₀ hydrocarbyl; R₃, R₄ are independently selected from H, C₁-C₁₀ hydrocarbyl, C₁-C₁₀ hydrocarbon carbonyl, C₁-C₁₀ hydrocarbon sulfonic group, C₁-C₁₀ hydrocarbon alcohol sulfonic group, C₁-C₁₀ hydrocarbon carboxylic group or -SO₃(M)ₙ; -(Polyoxyalkylene)₁- is one or more of -(PO)ₓ₁-, -(EO)_{y1}- and -(BO)_{z1}-; -(Polyoxyalkylene)₂- is one or more of -(PO)ₓ₂-, -(EO)_{y2}- and - (BO)_{z2}-; x1+x2=0-30, y1+y2=1-30, z1+z2=0-30; M is selected from any one of alkali metal ion and alkaline earth metal ion; when M is an alkali metal ion, n is 1, and when M is an alkaline earth metal ion, n is 0.5.

6. The hydrocarbyl tetralin polyethersulfonate according to claim 1, **characterized in that** in formula (I) R₁ is C₈-C₁₆ alkyl, preferably C₈-C₁₂ alkyl; and/or
R₂ is H or C₁-C₁₂ alkyl, preferably H or C₁-C₈ alkyl; and/or
R₃, R₄ are independently selected from H, C₁-C₁₀ alkyl, C₁-C₁₀ alkyl carbonyl, C₁-C₁₀ alkyl sulfonic group, C₁-C₁₀ alkyl alcohol sulfonic group, C₁-C₁₀ alkyl carboxylic group or -SO₃(M)ₙ; preferably, R₃, R₄ are independently selected from H, C₁-C₆ alkyl or -SO₃(M)ₙ; more preferably, R₃, R₄ are independently selected from H, -CH₃, -CH₂CH₃ or -SO₃(M)ₙ.

7. The hydrocarbyl tetralin polyethersulfonate according to any one of the preceding claims, **characterized in that** z1 and z2 are zero, x1 +x2=0-12, y1+y2=4-20; preferably, z1 and z2 are zero, x1+x2=0-8, y1+y2=4-12; more preferably, z1 and z2 are zero, x1+x2=0-4, y1+y2=4-8; and/or
z1 and z2 are zero, x1+x2+y1+y2=1-50, preferably 5-20, more preferably 5-15;
M is selected from any one of sodium ion, potassium ion, calcium ion, and magnesium ions and/or
X is N.

8. The hydrocarbyl tetralin polyethersulfonate according to any one of claims 1-6, **characterized in that** z1 and z2 are zero, x1+x2=1-6, y1+y2=4-8; and X is N.

9. The hydrocarbyl tetralin polyethersulfonate according to any one of claims 1-6, **characterized in that** x1+x2=1-6, y1+y2=4-8, z1+z2=1-6;
X is N; and/or
-N-(Polyoxyalkylene₁)- and -N-(Polyoxyalkylene₂)- are each arranged in the way of -N-BO_{z1}-POₓ₁-EO_{y1}- or -N-BO_{z2}-POₓ₂-EO_{y2}-.

10. A method for preparing the hydrocarbyl tetralin polyethersulfonate according to claims 1-9, comprising the steps of:
S100. reacting the hydrocarbyl tetralin shown by formula (II-0) with a nitration reagent to produce hydrocarbyl nitrotetralin; 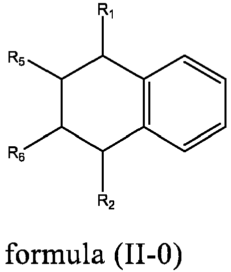
in formula (II-0), R₁, R₂, R₅ and R₆ are defined according to any one of claims 1-7;
S200. hydrotreating the hydrocarbyl nitrotetralin obtained in step S100 to obtain alkyl tetralin amine;
S300. reacting the hydrocarbyl tetralin amine obtained in step S200 with one or more of epoxyethane, epoxypropane and epoxybutane to obtain hydrocarbyl tetralin amino polyether of formula (I-0);
S400. reacting the hydrocarbyl tetralin amino polyether obtained in step S300 with a sulfonation reagent to obtain a sulfonated product which is hydrolyzed to obtain hydrocarbyl tetralin polyethersulfonate of formula (I-0).

11. The method according to claim 10, **characterized in that** the nitration reagent in step S100 is at least one of nitric acid and dinitrogen pentoxide, or a mixed acid composed of at least one of nitric acid and dinitrogen pentoxide and at least one selected from concentrated sulfuric acid, glacial acetic acid, acetic anhydride and phosphorus pentoxide;
preferably, in the step S100, the molar ratio of the hydrocarbyl tetralin to the nitration reagent is 1:1-3; the reaction temperature is preferably 0-80°C; the reaction time is preferably 1-10 hours; more preferably, the reaction temperature is 20-60°C and the reaction time is 1-3 hours;
preferably, the hydrotreatment in step S200 is carried out at a temperature of 20-150°C, preferably 50-70°C, and a pressure of less than 4 MPa, for example 1-3 MPa.

12. The method according to claim 10 or 11, **characterized in that** in step S300, the molar ratio of the epoxypropane to the hydrocarbyl tetralin amine is 0-30:1, preferably 0-12:1, more preferably 0-8:1, further preferably 0-4:1; the molar ratio of the epoxyethane to the hydrocarbyl tetralin amine is 1-30:1, preferably 4-20:1, more preferably 4-12:1, further preferably 4-8:1; the molar ratio of the epoxybutane to the hydrocarbyl tetralin amine is 0-30:1, preferably 0; and/or
in step S300, the amount of the epoxybutane is zero, and the molar ratio of the total amount of the epoxypropane and epoxyethane to the hydrocarbyl tetralin amine is 1-50:1, preferably 5-20:1, more preferably 5-15:1; and/or
in step S300, the reaction temperature is 135-200°C, preferably 140-180°C, more preferably 140-160°C; the pressure is 0-5 MPa, preferably 0-3 MPa, more preferably 0-0.4 MPa.

13. The method according to any one of claims 10-12, **characterized in that** the step S400 includes:
S401. reacting the hydrocarbyl tetralin amino polyether with the sulfonation reagent in a molar ratio of 1:1-5 at a temperature of 20-80°C for 0.5-10 hours to obtain a sulfonated product;
S402. adjusting the pH value of the sulfonated product obtained in step S401 to 10-14 and hydrolyzing for 0.5-5 hours to obtain the hydrocarbyl tetralin polyethersulfonate.

14. A surfactant composition, comprising the hydrocarbyl tetralin polyethersulfonate according to any one of claims 1-9.

15. An oil displacing agent composition, comprising the hydrocarbyl tetralin polyethersulfonate according to any one of claims 1-9 and water, wherein the weight ratio of the hydrocarbyl tetralin polyethersulfonate to water is preferably 1:(50-2000), more preferably 1:(100-1000).

16. Use of said hydrocarbyl tetralin polyethersulfonate according to any one of claims 1-9 or said oil displacing agent composition according to claim 15 in an oil reservoir exploitation.
